# EUROPEAN PATENT APPLICATION

(11) **EP 4 818 193 A1**
(43) Date of publication of application: **07.10.2026**
(21) Application number: 24896792.9
(22) Date of filing: 02.12.2024
(51) Int. Cl.: C07K 7/64, C12P 21/04, A61K 38/12, A61P 9/10, A61P 25/00

(54) **COMPOSITION AND METHOD FOR TREATING NEONATAL HYPOXIC-ISCHEMIC ENCEPHALOPATHY**

(30) Priority: 01.12.2023 CN 202311635832
(71) Applicant: Suzhou Glenkol Pharma Technology Co., Ltd., Suzhou, Jiangsu, 215127 (CN); Quanzhou Haichuang Pharmaceutical Technology Co., Ltd., Quanzhou, Fujian 362221 (CN); Huiankai (Xiamen) Pharmaceutical Technology Co., Ltd., Xiamen, Fujian 361028 (CN)
(72) Inventor: CHEN, Jinhui, Suzhou, Jiangsu 215127 (CN)
(74) Representative: Hautier IP - MC/EP
(86) International application number: PCT/CN2024/136157
(87) International publication number: WO 2025/113713

(57) **Abstract**

Use of an effective amount of an active peptide in the manufacture of a medicament for the preventing and/or treating neonatal hypoxic-ischemic encephalopathy, wherein the active peptide has an amino acid sequence comprising KKNRNKLRRQHSY or a functionally equivalent variant thereof. Optionally, the active peptide further comprises a signal peptide at a N-terminus or C-terminus thereof.

## Description

The present disclosure claims priority to Chinese Patent Application CN202311635832.6, filed on December 1, 2023, the disclosure of which is hereby incorporated by reference in its entirety.

### TECHNICAL FIELD

The present disclosure relates to the field of research and development of small-molecule polypeptide drugs, particularly to the use of an effective amount of an active peptide in the preparation of a meidcament for preventing and/or treating neonatal hypoxic-ischemic encephalopathy.

### BACKGROUND

Neonatal hypoxic-ischemic encephalopathy (HIE) is a hypoxic-ischemic brain injury resulting from fetal asphyxia during the prenatal, perinatal, or postnatal periods. Common pathogenic factors include fetal intrauterine distress caused by various reasons (approximately 50%), such as umbilical cord around the neck and abnormal amniotic fluid. HIE is also frequently caused by asphyxia and hypoxia during delivery (approximately 40%) and congenital diseases (approximately 10%). The incidence of neonatal hypoxic-ischemic encephalopathy is 1 to 8 cases per 1,000 neonates in developed countries, while in developing countries, the incidence is as high as 26 cases per 1,000 neonates. WHO data shows that globally, between 2000 and 2003, approximately 848,000 children under the age of 5 died annually from neonatal asphyxia, i.e., neonatal hypoxic-ischemic encephalopathy. 40% to 60% of infants with neonatal hypoxic-ischemic encephalopathy die before the age of 2, or suffer from sequelae such as intellectual retardation, epilepsy, and cerebral palsy.

Currently, there are no clinical drugs available to inhibit neuronal cell death caused by neonatal hypoxic-ischemic encephalopathy. The only proven effective treatment is mild hypothermia therapy for neonates. Typically, targeted cranial cooling is adopted to reduce the head temperature of pediatric patients to 34.5°C, or whole-body cooling to 33.5°C. The hypothermia treatment lasts for 48 to 72 hours, followed by gradual rewarming back to 36.5°C at a rate of 0.5° C per hour. Temperature control and treatment duration for hypothermia therapy are subject to strict requirements. At temperatures as low as 32°C, the neuroprotective effect is lost, while at temperatures below 30°C, severe injury can actually be caused. Furthermore, hypothermia therapy should be initiated within 6 hours of the onset of neonatal hypoxic-ischemic encephalopathy; otherwise, the treatment will be ineffective. The therapeutic time window is narrow. Consequently, even among infants who receive hypothermia therapy, less than half achieve symptomatic improvement.

Although certain drugs have demonstrated therapeutic effects in inhibiting neuronal cell death in adults with neurological injuries in recent years, neonatal hypoxic-ischemic encephalopathy differs from neurological injuries such as ischemic stroke and traumatic brain injury in adults in terms of pathogenesis, pathological processes and treatment strategies. Consequently, these drugs have not shown clear therapeutic efficacy in neonatal hypoxic-ischemic encephalopathy.

The primary cause of traumatic brain injury is mechanical injury to neurons and nerve fibers induced by external force, and current treatment strategies focus on controlling intracranial pressure and traumatic hemorrhage. In contrast, neonatal hypoxic-ischemic encephalopathy is primarily caused by energy exhaustion resulting from oxygen and glucose deprivation, which is completely distinct from the pathogenic mechanism of traumatic brain injury. Consequently, while hypothermia therapy has demonstrated clear therapeutic efficacy for neonatal hypoxic-ischemic encephalopathy, its effectiveness in treating adult traumatic brain injury remains highly controversial.

Compared to ischemic stroke in adults, although both conditions arise from ischemia and hypoxia, hypoxia and ischemia in adult stroke are primarily caused by local vascular occlusion due to thrombosis, leading to focal brain tissue injury around the thrombus. Corresponding treatment includes thrombolysis, endovascular therapy, and antiplatelet medications. In contrast, neonatal hypoxic-ischemic encephalopathy primarily results from asphyxia during the birthing process, causing widespread hypoxia and ischemia that affects the entire brain. Furthermore, compared to adults, neonatal brain tissue has a higher metabolic rate and is more suseptible to hypoxia and ischemia. With limited glycogen stores in the brain and high reliance on blood circulation, neonates are prone to hypoglycemia and disorders of normal nervous system metabolism under hypoxic-ischemic conditions. Furthermore, the neonatal brain is characterized by fragile cerebral blood vessels and immature cerebral autoregulation, resulting in poorer tolerance to ischemia and hypoxia. Consequently, the brain insult suffered in neonatal hypoxic-ischemic encephalopathy is more intense. More importantly, the neonatal brain is still in the developmental stage. Compared to the adult brain, neonatal neurons have not yet fully differentiated and matured, and exhibit significant differences from adult neurons in terms of receptor expression, cell morphology and structure, synaptic density, axonal myelination, cellular excitability, and susceptibility to cell death. Consequently, it is difficult to predict the efficacy of therapeutic approaches or drugs effective for ischemic stroke in adults against neonatal hypoxic-ischemic encephalopathy.

In summary, the drug development for neonatal hypoxic-ischemic encephalopathy shall take the specific disease characteristics into full consideration. Accordingly, there remains an urgent need in the art for drugs capable of effectively inhibiting neuronal cell death and protecting neonatal brain tissue in cases of neonatal hypoxic-ischemic encephalopathy.

### SUMMARY

The present disclosure provides the use of an effective amount of an active peptide in the manufacture of a medicament for preventing and/or treating neonatal hypoxic-ischemic encephalopathy.

In an embodiment, the present application provides a method for preventing and/or treating neonatal hypoxic-ischemic encephalopathy, comprising administering to a subject an effective amount of the active peptide according to the present disclosure. In an embodiment, administration of an effective amount of the active peptide according to the present disclosure to a subject inhibits at least one neural cell death in the subject.

In an embodiment, the present application provides a method for preventing and/or inhibiting neural cell injury caused by neonatal hypoxic-ischemic encephalopathy, comprising exposing at least one neural cell of a neonatal subject to an effective amount of the active peptide, thereby inhibiting the injury of the at least one neural cell in the subject.

In an embodiment, the step of exposing at least one neural cell to an effective amount of the active peptide according to the present invention or administering an effective amount of the active peptide according to the present invention to a subject is performed within a short period of time after hypoxic-ischemic encephalopathy. In an exemplary embodiment, the active peptide may be administered within a period of time following neonatal hypoxic-ischemic encephalopathy, such as within one month, two weeks, one week, 72 hours, 48 hours, 24 hours, 18 hours, 12 hours, 8 hours, 6 hours, 4 hours, 2 hours, and 1 hour; preferably within two weeks, one week, or 72 hours; more preferably within 48 hours; and even more preferably within 24 hours or less, for example, within 18, 12, 8, 6, 4, 2, or 1 hour.

In an embodiment, the present disclosure provides use of an effective amount of the active peptide in the manufacture of a medicament for inhibiting neural cell injury following neonatal hypoxic-ischemic encephalopathy.

In an embodiment, the present disclosure provides use of an effective amount of the active peptide in the manufacture of a medicament for inhibiting neural cell injury at the acute phase of neonatal hypoxic-ischemic encephalopathy.

In another embodiment, the present disclosure provides use of an effective amount of the active peptide in the manufacture of a medicament for treating a subject with neonatal hypoxic-ischemic encephalopathy. The subject may be a mammal, such as a human.

In another embodiment, the present disclosure provides a composition for treating neural cell injury following neonatal hypoxic-ischemic encephalopathy, wherein the composition comprises the active peptide according to the present disclosure and a pharmaceutically acceptable carrier.

In another embodiment, the present disclosure provides the active peptide according to the present disclosure or a composition comprising the active peptide according to the present disclosure, for use in preventing and/or treating neonatal hypoxic-ischemic encephalopathy.

In another embodiment, the present disclosure provides the active peptide according to the present disclosure or a composition comprising the active peptide according to the present disclosure, for use in inhibiting neural cell injury following neonatal hypoxic-ischemic encephalopathy.

In another embodiment, the present disclosure provides the active peptide according to the present disclosure or a composition comprising the active peptide according to the present disclosure, for use in inhibiting neural injury at the acute phase of neonatal hypoxic-ischemic encephalopathy.

In another embodiment, the present disclosure provides the active peptide according to the present disclosure or comprising the active peptide according to the present disclosure, for use in preventing and/or treating neonatal hypoxic-ischemic encephalopathy.

In another embodiment, the present disclosure provides the active peptide according to the present disclosure or comprising the active peptide according to the present disclosure, for use in inhibiting neural cell injury following neonatal hypoxic-ischemic encephalopathy.

In another embodiment, the present disclosure provides the active peptide according to the present disclosure or comprising the active peptide according to the present disclosure, for use in inhibiting neural injury at the acute phase of neonatal hypoxic-ischemic encephalopathy.

In another embodiment, the active peptide according to the present disclosure is formulated into a dosage form for gastrointestinal or parenteral administration, wherein the dosage form is a pharmaceutically acceptable oral dosage form, injectable dosage form, or powder for injection.

In another embodiment, the therapeutically effective amount of the active peptide according to the present disclosure ranges from 0.1 to 1000 mg/kg body weight, for example 1 to 100 mg/kg body weight, or 10 to 50 mg/kg body weight.

In another embodiment, the peptide according to the present disclosure may comprise SEQ ID NO: 1 (KKNRNKLRRQHSY) or a functionally equivalent variant thereof, wherein the variant has 1, 2, or 3 conservative amino acid substitutions, additions, or deletions. The peptides according to the present disclosure may be selected from sequences having at least 80%, at least 85%, at least 90%, at least 95%, at least 99%, or 100% identity to SEQ ID NO: 1.

In another embodiment, the peptides according to the present disclosure may further comprise a signal peptide at the N-terminus or C-terminus thereof. The signal peptide is selected from a TAT sequence, a MAP sequence, a MTS sequence, or an R9 sequence.

In another embodiment, the active peptides according to the present disclosure may be chemically modified.

Without being limited by theory, the inventors of the present disclosure have discovered that the active peptides according to the present disclosure can surprisingly inhibit neurological injury following neonatal hypoxic-ischemic encephalopathy, significantly enhance brain tissue survival following neonatal hypoxic-ischemic encephalopathy, and improve the overall health status of neonates.

### BRIEF DESCRIPTION OF DRAWINGS

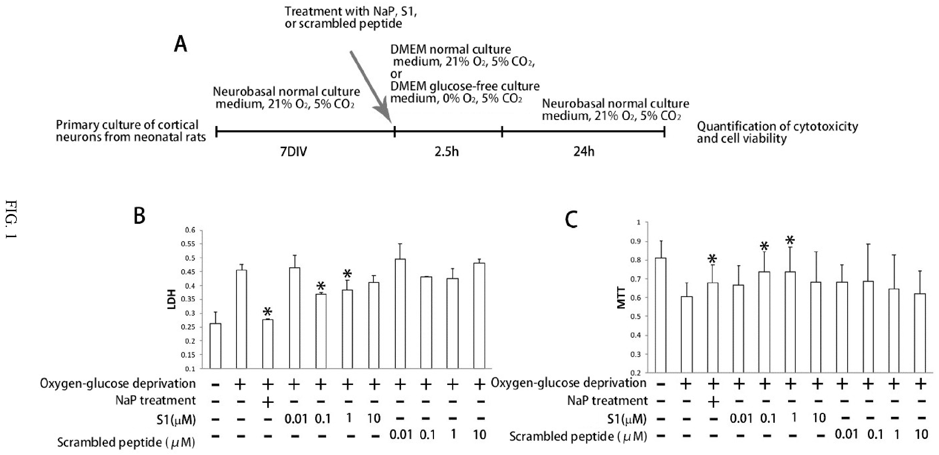

FIG. 1 illustrates that pretreatment with S1 in vitro reduces neural cell injury caused by oxygen-glucose deprivation, wherein:
   Panel 1A shows experimental procedures for evaluating the neuroprotective effects of S1 pretreatment against excitotoxic injury following oxygen-glucose deprivation;
   Panel 1B shows lactate dehydrogenase (LDH) assay for cytotoxicity, quantitatively demonstrating the neuroprotective effect of S1 pretreatment; and
   Panel 1C shows MTT assay for cell viability, quantitatively demonstrating the neuroprotective effect of S1 pretreatment.

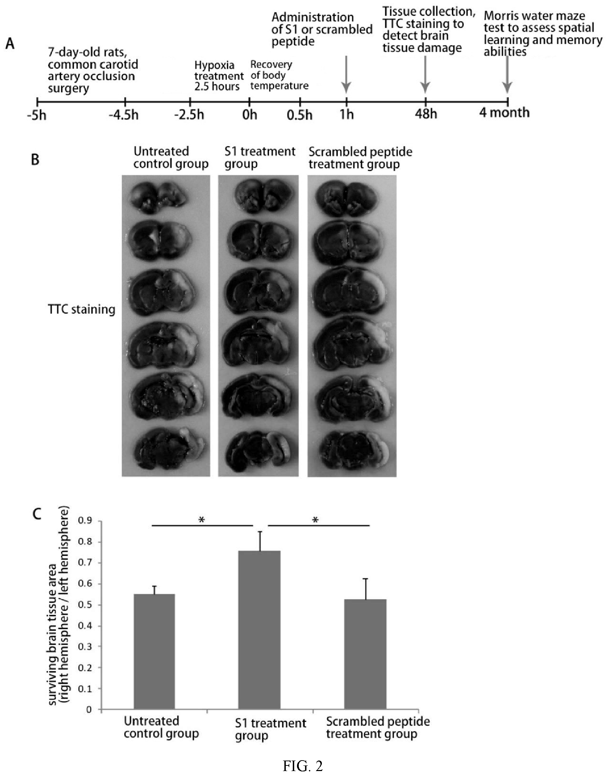

FIG. 2 illustrates that S1 treatment significantly improves brain tissue survival in neonatal rats following neonatal hypoxic-ischemic encephalopathy, wherein:
   Panel 2A shows experimental procedures for evaluating the neuroprotective effects of S1 treatment against neonatal hypoxic-ischemic encephalopathy in neonatal rats;
   Panel 2B shows brain tissue imaging after TTC staining in neonatal rats treated with S1 or a scrambled peptide following neonatal hypoxic-ischemic encephalopathy injury; and
   Panel 2C shows quantification of surviving brain tissue.
FIG. 3 illustrates that S1 treatment significantly improves body weight gain in neonatal rats following neonatal hypoxic-ischemic encephalopathy injury, especially shows the quantification of body weight gain relative to pre-injury levels in neonatal rats treated with S1 or the scrambled peptide at 24 and 48 hours after hypoxic-ischemic encephalopathy.

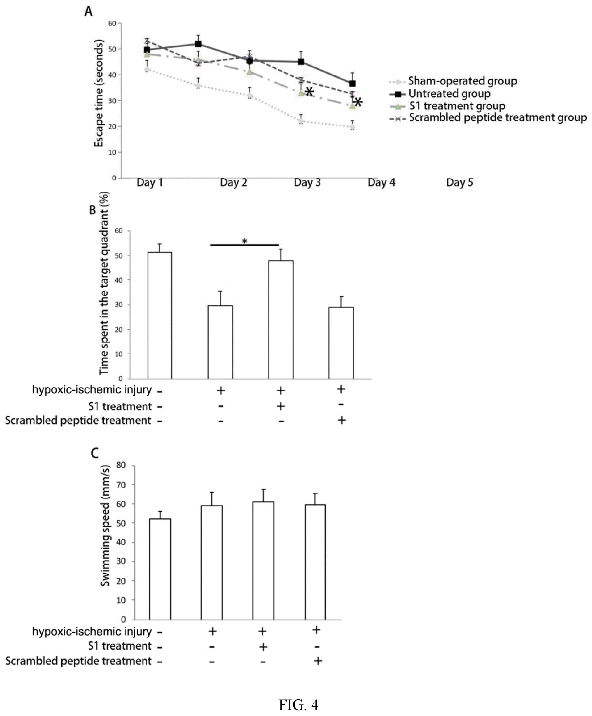

FIG. 4 illustrates that S1 treatment significantly improves spatial learning and memory abilities in adult rats following neonatal hypoxic-ischemic encephalopathy, wherein:
   Panel 4A shows the time spent by rats to reach the underwater platform in the morris water maze on different days of training to assess spatial learning ability, wherein, neonatal rats were treated with S1 or a scrambled peptide following hypoxic-ischemic encephalopathy injury, and the test was performed 4 months after the injury;
   Panel 4B shows, on day 6 of the morris water maze after platform removal, the swimming time spent by rats in the target quadrant where the platform had previously been located, to assess spatial memory ability in rats; and
   Panel 4C shows the swimming speed of rats measured in the morris water maze.

### DETAILED DESCRIPTION

For the purpose of facilitating the understanding of the principles of the present disclosure, embodiments are described with reference to the accompanying drawings. These descriptions are not intended to limit the scope of the present disclosure.

The present disclosure provides various compositions, methods, and uses for treating neural injury. In various embodiments of the present disclosure, the injury may be caused by neonatal hypoxic-ischemic encephalopathy.

As used herein, the term "mammal" refers to any animal classified as a mammal, including humans, domestic and farm animals, as well as zoo animals, sports animals, and pet animals, such as dogs, cats, cattle, horses, sheep, pigs, goats, rabbits, and the like.

As used herein, the term "therapeutically effective amount" refers to an amount of a molecule, peptide, or other drug effective in "treating" neurological impairment or injury in a subject or mammal. In the case of neonatal hypoxic-ischemic encephalopathy, a therapeutically effective amount of the "drug" may reduce the level of neural cell death or injury; reduce the area of damaged neural tissue; and/or alleviate, to a certain extent, one or more symptoms associated with neonatal hypoxic-ischemic encephalopathy. In one embodiment, the therapeutically effective amount includes, but is not limited to, about 0.1-1000 mg/kg body weight, for example 1-100 mg/kg body weight or 10-50 mg/kg body weight.

Method for Inhibiting Neurological Injury:
In at least one embodiment of the present disclosure, neurological injury may be treated by delivering an inhibitor to the brain. For example, the method for inhibiting neural injury according to the present disclosure may include: exposing at least one neural cell to an effective amount of an active peptide, thereby inhibiting injury of the at least one neural cell. Furthermore, the drug for use in the method may comprise a peptide, a nucleotide, or a molecule. Optionally, the drug may also comprise a pharmaceutically acceptable carrier.

According to the present disclosure, provided is a method for treating a subject suffering from traumatic brain injury. The method includes administering a therapeutically effective amount of an active peptide to the subject, thereby inhibiting injury of at least one neural cell in the subject. In at least one embodiment, the subject may be a mammal, such as a human. Furthermore, administration of the therapeutically effective amount of the peptide may be performed via the following routes: oral administration, intravenous injection, intramuscular injection, subcutaneous injection, retrograde intravenous injection, arterial injection, transdermal administration, inhalation and surgical implantation. In an exemplary embodiment, the method for treating a subject with neonatal hypoxic-ischemic encephalopathy according to the present disclosure reduces the level of brain tissue damage and improves spatial learning and memory abilities of the subject.

According to the method of the present disclosure, the step of exposing at least one neural cell to an effective amount of the peptide according to the present disclosure or administering an effective amount of the peptide according to the present disclosure to a subject is performed within a short period of time following neonatal hypoxic-ischemic encephalopathy. In an exemplary embodiment, the administration of the active peptide according to the present disclosure is performed within one week after neonatal hypoxic-ischemic encephalopathy injury, preferably within 72 hours, more preferably within 48 hours, and even more preferably within 24 hours or less, for example, within 18, 12, 8, 6, 4, 2, or 1 hour.

The peptides according to the present disclosure:
In an exemplary embodiment, peptides suitable for the present disclosure include a peptide having the sequence of SEQ ID NO: 1 (KKNRNKLRRQHSY) or a sequence having at least about 80%, at least about 85%, at least about 90%, at least about 95%, at least about 99%, or 100% identity to SEQ ID NO: 1.

In an exemplary embodiment, peptides suitable for the present disclosure include functionally equivalent variants of a peptide having SEQ ID NO: 1 or consisting of SEQ ID NO: 1. For example, the variant has 1, 2, or 3 conservative amino acid substitutions, additions, or deletions and exhibits in vivo or in vitro activity substantially similar to that of the exemplary peptides according to the present disclosure.

Variants of the peptides disclosed herein are also within the scope of the present disclosure. A peptide variant refers to an amino acid sequence in which one or more amino acids have been altered. A variant may have "conservative" changes, wherein the substituted amino acid has similar structural or chemical properties, such as replacing leucine with isoleucine. Alternatively, a variant may have "non-conservative" changes, such as replacing glycine with tryptophan. Similar minor changes may also include amino acid deletions or insertions, or a combination of both. A specific form of a "variant" peptide is a "functionally equivalent" peptide, i.e., a peptide that exhibits in vivo or in vitro activity substantially similar to that of the exemplary peptides disclosed herein. Guidance for determining which amino acid residues may be substituted, inserted, or deleted without loss of biological or immunological activity may be found using computer programs well known in the art, such as DNASTAR software (DNASTAR, Inc., Madison, WI). In addition, specific guidance is provided below, including that set forth in the cited references, which are incorporated herein by reference in their entirety.

In further embodiments, the specific positions of the named residues may vary slightly while still occupying structurally and functionally analogous positions within the peptide (see Chang, Y., et al., Biochemistry 37:3258-3271 (1998)).

Furthermore, the peptide according to the present disclosure may further include, at its N-terminus or C-terminus, a signal peptide capable of guiding the peptide according to the present disclosure across the blood-brain barrier and neural cell membranes. Suitable signal peptides known in the art may be used in the present disclosure, provided that they are capable of guiding the peptide of the present invention across the blood-brain barrier and neural cell membranes. Exemplary signal peptides may be selected from a TAT sequence ("GRKKRRQRRR (SEQ ID NO: 3)", "YGRKKRRQRRR (SEQ ID NO: 4)", "YGRKKRRQRRRPPQ (SEQ ID NO: 5)" or "GRKKRRQRRRQ (SEQ ID NO: 6)"), a model amphipathic peptide (MAP) sequence ("KLALKLALKALKAALKLA (SEQ ID NO: 7)"), a membrane translocating peptide (MTS) sequence ("AAVALLPAVLLALLAP (SEQ ID NO: 8)") or an R9 sequence ("RRRRRRRRR (SEQ ID NO: 9)"). In one embodiment, the signal peptide disclosed herein is the Tat sequence. The peptide according to the present disclosure may be chemically modified to facilitate its penetration across the BBB and neural cell membranes, including but not limited to amidation, acetylation, or cyclization. In an exemplary embodiment, the peptide according to the present disclosure includes or consists of the sequence YGRKKRRQRRRKKNRNKLRRQHSY (SEQ ID NO: 2), which is designated as S1.

The scrambled peptide according to the present disclosure is Tat-NRRRNSKLQHKKY (SEQ ID NO: 10), and the full sequence thereof is YGRKKRRQRRRNRRRNSKLQHKKY.

In an exemplary embodiment, the peptide according to the present disclosure is a short-chain peptide with strong hydrophobicity, enabling it to readily cross the blood-brain barrier and neural cell membranes with the aid of a signal peptide.

### Examples

The following examples are provided merely to illustrate the present disclosure and are not intended to limit it.

In the present disclosure, the inventors evaluated the neuroprotective effects of brainpenetrating peptides in inhibiting cellular excitotoxicity, improving neural survival, reducing brain tissue injury, and improving overall health status and behavioral outcomes following neonatal hypoxic-ischemic encephalopathy.

Example 1: In in vitro experiments, S1 can protect neurons from cellular excitotoxicity induced by oxygen-glucose deprivation through pretreatment.

This example is designed to assess the neuroprotective effects of S1 against excitotoxic injury following oxygen-glucose deprivation and to determine the optimal dose of S1 in neural cells in vitro.

Culture of cerebral cortical neural cells: The brains of neonatal rats were dissected, the meninges removed, and the cerebral cortex isolated. The cortical tissues were digested with papain, single cells collected, and plated onto lysine-treated coverslips and cultured in Neurobasal^{™} serum-free medium. To mimic hypoxic-ischemic encephalopathy injury in vitro, the Neurobasal medium was replaced with DMEM medium at 7 days in vitro (DIV7) and cells were cultured under 21% O₂/5% CO₂ for 2.5 hours to serve as the control group, or replaced with glucose-free DMEM medium and cultured under 0% O₂/5% CO₂ for 2.5 hours to serve as the oxygen-glucose deprivation treatment group. After treatment, both groups were returned to Neurobasal medium and cultured normally under 21% O₂/5% CO₂ for 24 hours.

S1 Pretreatment: At the start of the oxygen-glucose deprivation treatment, cells were simultaneously treated with S1 (final concentrations of 0.01 M, 0.1 M, 1 M, or 10 M), or with the scrambled peptide Tat-NRRRNSKLQHKKY (SEQ ID NO: 10), or with NaP (Davunetide) as a control.

Lactate dehydrogenase (LDH) kit cytotoxicity assay: After 24 hours of normal culture, collect the cell culture supernatant from each group, add the LDH assay working solution, mix thoroughly, and incubate at room temperature in the dark for 30 minutes. Then, measure the absorbance at 490 nm and calculate the LDH release level, which indicates cytotoxicity.

MTT assay for cell viability: After 24 hours of normal culture, MTT solution was added to each group and cultured for an additional 4 hours. After terminating the culture, the culture medium was discarded, and DMSO was added followed by low-speed shaking for 10 minutes. Then, measure the absorbance at 490 nm and calculate the MTT formation level, which indicates cell viability.

### Results

To evaluate the effects of S1 pretreatment on neural survival under oxygen-glucose deprivation-induced injury, NaP (final concentration 2 mM) or S1 (final concentrations 0.01 M, 0.1 M, 1 M, or 10 M) or scrambled peptides (final concentrations 0.01 M, 0.1 M, 1 M, or 10 M) were added to the cell culture medium at the initiation of oxygen-glucose deprivation treatment. At 24 hours after injury, LDH and MTT assays were performed to assess excitotoxicity and neural survival (Panel 1A). Following oxygen-glucose deprivation treatment, the untreated group exhibited increased LDH release levels and decreased MTT levels, indicating that oxygen-glucose deprivation induced neural toxicity and reduced neural survival. As a neuroprotection positive control, treatment with 2 mM NaP significantly reduced LDH release levels (p < 0.05, Panel 1B) and significantly increased MTT formation levels (p < 0.05, Panel 1C) compared to the untreated oxygen-glucose deprivation group, demonstrating neuroprotective effects. S1 treatment at concentrations of 0.1 M and 1 M significantly reduced LDH release levels (p < 0.05, Panel 1B) and significantly increased MTT formation levels (p < 0.05, Panel 1B) compared to the untreated oxygen-glucose deprivation group, indicating that S1 pretreatment reduces excitotoxicity and improves neural survival. In contrast, treatment with the scrambled peptide at any concentration did not alter LDH release levels or MTT formation levels compared to the untreated group (Panel 1B and 1C). Therefore, S1 pretreatment at concentrations of 0.1-1 M exerts neuroprotective effects on cells in vitro.

Example 2: In in vivo experiments, S1 protects brain tissue from neonatal hypoxic-ischemic encephalopathy, improves the animals' overall health following injury, and rescues their spatial learning and memory abilities of animals in adulthood.

This example is designed to evaluate the neuroprotective effects of S1 following neonatal hypoxic-ischemic encephalopathy, as well as its effects on improving the overall health status of injured animals and their spatial learning and memory abilities in adulthood.

Animal model of neonatal hypoxic-ischemic encephalopathy and S1 treatment (Panel 2A): Seven-day-old neonatal rats underwent unilateral common carotid artery occlusion. After 2 hours of postoperative recovery, the rats were placed in a hypoxic environment containing 8% oxygen and 92% nitrogen for 2.5 hours. One hour after the completion of hypoxia treatment, S1 or the scrambled peptide (10 mg/kg body weight) was administered via tail vein injection.

Brain tissue injury assessment with TTC: Animals were euthanized at 48 hours after the hypoxia treatment, and their brains were harvested, sectioned, and incubated in TTC solution at 37°C for 30 minutes. Then the brain sections were photographed under an optical microscope, and the necrotic area of brain tissue was measured to calculate the area of surviving brain tissue.

Body weight measurement to assess overall health status: Body weights of neonatal rats were measured respectively before surgery, at 24 hours and 48 hours after neonatal hypoxic-ischemic encephalopathy (HIE) injury. The body weight growth rate after surgery relative to the preoperative level was calculated.

Morris water maze test for spatial learning and memory: Four months after the completion of hypoxia treatment, rats underwent a 5-day water maze training session to learn to locate a submerged platform. The time taken to reach the platform was recorded to calculate learning scores. On the day following the completion of the training, a probe trial was conducted to evaluate spatial memory. During the probe trial, the platform was removed, and the total swimming distance of rats within the target quadrant was recorded. Data were plotted as a learning curve chart showing the time to reach the platform during the five-day water maze testing. The total time spent in the target quadrant within the 60-second probe trial was also recorded. Additional documented variables included swimming speed. The cumulative latency in the target quadrant and the original platform area was further measured for the calculation of memory scores.

### Results:

To evaluate the effects of treatment with S1 or the scrambled peptide on brain tissue survival following neonatal hypoxic-ischemic encephalopathy injury, S1 (final concentration 10 mg/kg body weight) or the scrambled peptide was administered via tail vein injection 1 hour after injury. At 48 hours post injury, brain tissue was stained with TTC. Healthy brain tissue reduces TTC and stains red, whereas damaged brain tissue loses the capacity to reduce TTC and therefore does not stain red, appearing white (Panel 2B). In the untreated control group, neonatal hypoxic-ischemic injury caused significant brain tissue necrosis, with large areas of brain tissue appearing white and visible damage on the brain surface; the survival rate of brain tissue was only 54.9% (Panel 2B, 2C). In the S1 treatment group, the area of white necrotic brain tissue was significantly reduced compared to the untreated group, and surface damage was not prominent; the brain tissue survival rate increased to 75.4%, significantly higher than that of the untreated injury group (p < 0.05, Panel 2B, 2C). In contrast, the area of white necrotic brain tissue in the scrambled peptide treatment control group was similar to that in the untreated injury group, with obvious surface damage to the brain tissue; the brain tissue survival rate was only 52.5%, significantly lower than that in the S1 treatment group (p < 0.05, Panel 2B and 2C). In conclusion, S1 treatment significantly improves brain tissue survival rate following neonatal hypoxic-ischemic encephalopathy and exerts a neuroprotective effect.

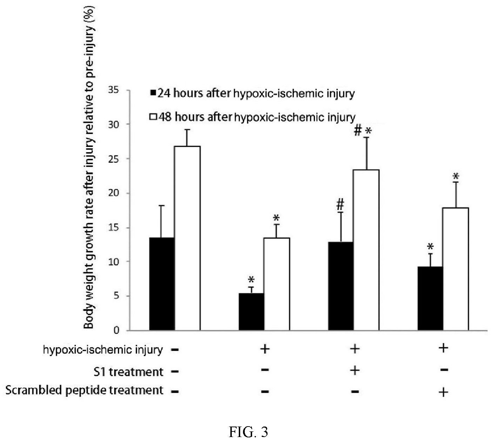

To evaluate the effects of S1 or scrambled peptide treatment on the overall health status of animals following neonatal hypoxic-ischemic encephalopathy injury, 7-day-old neonatal rats were weighed and their body weights recorded prior to injury, followed by the aforementioned injury treatments. S1 (final concentration 10 mg/kg body weight) or the scrambled peptide (final concentration 10 mg/kg body weight) was administered via tail vein injection at 1 hour after injury, and body weight was measured and recorded at 24 and 48 hours post-injury. In normal 7-day-old neonatal rats, body weight continued to increase over the following two days. Weight increased by 13.47% on day 8 and 26.77% on day 9 (FIG. 3). In contrast, neonatal rats that sustained neonatal hypoxic-ischemic encephalopathy injury on day 7 showed a weight gain of only 5.42% at 24 hours post-injury (day 8) and 13.4% on day 9, which was significantly lower than that of the sham-surgery control group (p < 0.05, FIG. 3). Therefore, neonatal hypoxic-ischemic encephalopathy severely impaired the overall health status of neonatal rats. In the S1 treatment group, body weight increased by 12.9% at 24 hours post-surgery (i.e., on Day 8) and by 23.32% at 48 hours post-injury (i.e., on Day 9). The body weight growth rates on both days were significantly higher than those in the untreated injury group (p < 0.05, FIG. 3). In contrast, neonatal rats in the scrambled peptide treatment group showed a body weight increase of only 9.23% at 24 hours post-injury (Day 8) and 17.89% at 48 hours post-injury (Day 9), which was significantly lower than that of the sham-surgery control group (p < 0.05, FIG. 3) and showed no statistically significant difference compared to the untreated injury group. Therefore, S1 significantly improves the overall health status of neonatal rats following neonatal hypoxic-ischemic encephalopathy injury.

To evaluate the effects of S1 or scrambled peptide treatment on spatial learning and memory abilities in adulthood following neonatal hypoxic-ischemic encephalopathy injury, 7-day-old neonatal rats received the aforementioned hypoxic-ischemic insult and underwent a 6-day Morris water maze test 4 months later. On the first training day, there were no significant differences in escape times among the groups, ensuring that the animals began the test at a similar behavioral baseline. In the sham-surgery group, escape times decreased daily over the first 4 days and reached the lowest level on day 5 (Panel 4A), indicating that they were learning the spatial location of the underwater platform. For the untreated injured rats, although their escape times also decreased gradually during training, they remained consistently longer than those of the sham-operated group, indicating that while they were also learning the spatial location of the underwater platform, their learning ability was impaired (Panel 4A). The learning curves of rats in the scrambled peptide treatment group were similar to those of the untreated injured rats, with no significant differences. This indicates that their impaired learning ability was not improved Panel 4A). In contrast, the escape times of rats in the S1 treatment group remained consistently shorter than those of the untreated injured group during training. Although they were still longer than those of the sham-operated group, they were significantly shorter than those of the untreated injured group on days 4 and 5 (p < 0.05), indicating that their spatial learning ability was improved compared to the untreated injured group (Panel 4A). Therefore, S1 treatment significantly improved the spatial learning ability of rats in adulthood following neonatal hypoxic-ischemic encephalopathy. In the probe trial on day 6, rats in the sham-surgery control group spent 51.16% of their time in the target quadrant, demonstrating their spatial memory of the underwater platform. However, the activity time in the target quadrant for rats in the untreated injury group decreased to 29.49%, indicating impaired spatial memory (Panel 4B). Rats in the S1 treatment group recovered their activity time in the target quadrant to about 47.86%, which was significantly higher than that of the untreated injury group (p < 0.05, Panel 4B), indicating a significant improvement in spatial memory ability. In contrast, rats in the scrambled peptide treatment group spent only 29.12% of their activity time in the target quadrant, showing no significant difference from the untreated injury group. In conclusion, S1 treatment significantly improved the spatial memory ability of rats in adulthood following neonatal hypoxic-ischemic encephalopathy. Swimming speed did not differ significantly among the groups, ruling out the possibility that swimming speed influenced behavioral performance.

Although various embodiments of the composition and method for treating neonatal hypoxic-ischemic encephalopathy injury have been described in detail herein, such embodiments are provided merely as non-limiting examples of the disclosure. Accordingly, those skilled in the art shall appreciate that various modifications and alterations can be made to the present disclosure without departing from the scope thereof. In fact, the present disclosure is not intended to be exhaustive or to limit its scope.

Furthermore, in the description of the representative embodiments, the methods and/or processes of the present disclosure have been set forth in a specific sequence of steps. However, such methods or processes should not be limited to the specific step sequence as described, and other step sequences are possible. Therefore, the specific sequence of steps disclosed herein should not be construed as a limitation on the scope of the present disclosure. Additionally, the disclosure regarding the methods and/or processes should not be limited to being carried out in the described sequence of steps. These sequences may vary and still fall within the scope of the present disclosure.

## Claims

1. Use of an effective amount of an active peptide in the manufacture of a medicament for preventing and/or treating neonatal hypoxic-ischemic encephalopathy, wherein the active peptide has an amino acid sequence comprising KKNRNKLRRQHSY (SEQ ID NO: 1) or a functionally equivalent variant thereof.

2. The use according to claim 1, wherein the amino acid sequence of the active peptide is KKNRNKLRRQHSY (SEQ ID NO: 1) or a functionally equivalent variant thereof.

3. The use according to claim 1 or 2, wherein the variant has 1, 2, or 3 conservative amino acid substitutions, additions, or deletions.

4. The use according to any one of claims 1 to 3, wherein the active peptide has a sequence having at least 80%, at least 85%, at least 90%, at least 95%, at least 99%, or 100% identity to KKNRNKLRRQHSY (SEQ ID NO: 1).

5. The use according to any one of claims 1 to 4, wherein the active peptide is chemically modified.

6. The use according to any one of claims 1 to 5, wherein the active peptide further comprises a signal peptide at a N-terminus or C-terminus of the active peptide.

7. The use according to claim 6, wherein the signal peptide is selected from a TAT sequence, a MAP sequence, a MTS sequence, or an R9 sequence.

8. The use according to claim 7, wherein the amino acid sequence of the TAT is selected from GRKKRRQRRR (SEQ ID NO: 3), YGRKKRRQRRR (SEQ ID NO: 4), YGRKKRRQRRRPPQ (SEQ ID NO: 5), or GRKKRRQRRRQ (SEQ ID NO: 6).

9. The use according to claim 8, wherein the amino acid sequence of the active peptide is YGRKKRRQRRRKKNRNKLRRQHSY (SEQ ID NO: 2).

10. The use according to any one of claims 1 to 9, wherein the active peptide is formulated as a medicament, and the medicament is administrated within a period of time following the onset of neonatal hypoxic-ischemic encephalopathy, said period of time being selected from one month, two weeks, one week, 72 hours, 48 hours, 24 hours, 18 hours, 12 hours, 8 hours, 6 hours, 4 hours, 2 hours, or 1 hour.

11. The use according to any one of claims 1 to 10, wherein the effective amount is in the range of 0.1 to 1000 mg/kg body weight.

12. The use according to any one of claims 1 to 11, wherein the active peptide is used to inhibit neural cell injury following neonatal hypoxic-ischemic encephalopathy.

13. The use according to any one of claims 1 to 11, wherein the neonatal hypoxic-ischemic encephalopathy is acute neonatal hypoxic-ischemic brain injury.

14. The use according to any one of claims 1 to 13, wherein the active peptide is formulated into a dosage form for gastrointestinal or parenteral administration.

15. The use according to claim 14, wherein the dosage form is a pharmaceutically acceptable oral dosage form, injectable dosage form, or powder for injection.
